# EUROPÄISCHE PATENTSCHRIFT

(11) **EP 0 876 819 B1**
(45) Veröffentlichungstag und Bekanntmachung des Hinweises auf die Patenterteilung: **20.08.2003**
(21) Anmeldenummer: 98107386.9
(22) Anmeldetag: 23.04.1998
(51) Int. Cl.: A61K 47/14, A61K 47/30

(54) **Verwendung von Copolymerisaten des N-Vinyl-pyrrolidons in Zubereitungen wasserunlöslicher Stoffe**
Use of copolymers of n-vinylpyrrolidone in the preparation of water insoluble compositions
Utilisation de copolymères de la n-vinylpyrrolidone pour la préparation de compositions insolubles

(30) Priorität: 07.05.1997 DE 19719187
(43) Veröffentlichungstag der Anmeldung: 11.11.1998
(73) Patentinhaber: BASF AKTIENGESELLSCHAFT, 67056 Ludwigshafen (DE)
(72) Erfinder: Kothrade, Stephan, Dr., 67117 Limburgerhof (DE); Meffert, Helmut, Dr., 67069 Ludwigshafen (DE); Berndl, Gunther, Dr., 67273 Herxheim (DE); Sanner, Axel, Dr., 67227 Frankenthal (DE); Stein, Stefan, Dr., 55286 Wörrstadt (DE); Schehlmann, Volker, Dr., 67354 Römerberg (DE); Ruchatz, Folker, Dr., 67433 Neustadt (DE)

(56) Entgegenhaltungen:
- DE-A- 3 842 820
- GB-A- 2 158 724
- US-A- 5 008 321
- US-A- 5 132 417

## Beschreibung

Die vorliegende Erfindung betrifft die Verwendung von Copolymerisaten aus
A) 60 bis 99 mol-% N-Vinylpyrrolidon, und
B) 1 bis 40 mol-% eines Monomeren aus gewählt aus der Gruppe der
   b1) C₈-C₃₀-Alkylester von monoethylenisch ungesättigten C₃-C₈-Carbonsäuren,
   b2) N-Alkyl- oder N,N-Dialkyl-substituierte Amide der Acrylsäure oder der Methacrylsäure mit C₈-C₁₈-Alkylresten,
   b3) Vinylester von aliphatischen C₈-C₃₀-Carbonsäuren, oder Gemischen dieser Monomeren,
   als oberflächenaktive Substanzen in Zubereitungen wasserunlöslicher Stoffe. Bevorzugt handelt es sich um die Verwendung in pharmazeutischen oder kosmetischen Zubereitungen.

In der DE-OS 25 14 100 sind Copolymere aus Vinylpyrrolidon und langkettigen Alkyl(meth)acrylaten sowie Terpolymere aus Vinylpyrrolidon, Vinylacetat und langkettigen Alkyl(meth)acrylaten zur Verwendung als Emulgatoren für Emulsionen vom Wasser in Öl-Typ beschrieben. Das Molverhältnis Vinylpyrrolidon zu Carbonsäureester in den beanspruchten Copolymeren liegt zwischen 1 : 1 und 1 : 20, vorzugsweise zwischen 1 : 1,5 und 1 : 10.

Die Verwendung der genannten Polymerisate als Emulgatoren in Emulsionen vom Öl- in- Wasser-Typ ist nicht bekannt. Die Verwendung von Co- und Terpolymeren mit einem Vinylpyrrolidon/ Carbonsäureester-Molverhältnis größer 1 : 1 in kosmetischen oder pharmazeutischen Zubereitungen ist ebenfalls nicht beschrieben.

Nicht immer sind pharmazeutische und kosmetische Wirkstoffe ausreichend wasserlöslich. Eine nicht ausreichende Wasserlöslichkeit von Wirkstoffen bedeutet, daß keine homogenen und aspektmäßig einwandfreien Zubereitungen erhalten werden und vielfach auch, daß die angestrebte Wirkung nicht optimal erreicht wird. Solche Wirkstoffe müssen deshalb mit amphiphilen Hilfsstoffen, z. B. Tensiden, solubilisiert werden. Auf diese Art und Weise werden die Bioverfügbarkeit und die Wirksamkeit des Wirkstoffes verbessert.

Aufgabe der Erfindung war es, Solubilisatoren für pharmazeutische, kosmetische oder andere wasserunlösliche Wirkstoffe oder Stoffe zur Verfügung zu stellen, die in der Lage sind, die Stoffe homogen zu dispergieren und somit in ausreichendem Umfang zur Absorption und Distribution im Organismus zur Verfügung zu stellen. Darüberhinaus sollten Substanzen bereit gestellt werden, die in der Lage sind, Öl in Wasser-Emulsionen zu stabilisieren.

Die Aufgabe wird erfindungsgemäß durch Verwendung der eingangs definierten Copolymerisate gelöst.

Die N-Vinylpyrrolidon enthaltenden Polymerisate werden dadurch hergestellt, daß man die entsprechenden Monomeren, die jeweils mindestens 60 Mol.-% Vinylpyrrolidon enthalten, radikalisch polymerisiert.

Als Comonomere B) kommen mindestens 1, höchstens 40 Mol.-%, bevorzugt 10 bis 20 MOl-% eines Monomeren ausgewählt aus einer der folgenden Gruppen b1) bis b3) oder Mischungen dieser Comonomeren in Betracht.

Geeignete Monomere b1) sind C₈-C₃₀-Alkylester von monoethylenisch ungesättigten C₃-C₈-Carbonsäuren. Geeignete Carbonsäuren sind beispielsweise Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure oder Itaconsäure, wobei Acrylsäure und/oder Methacrylsäure bevorzugt sind. Geeignete Alkylreste umfassen auch Cycloalkylreste. Bevorzugt werden Ester mit C₈-C₁₈-Alkylresten eingesetzt. Geeignete Monomere sind beispielsweise Octylacrylat, 2-Ethylhexylacrylat, 2-Ethylhexylmethacrylat, Nonylacrylat, Decylacrylat, Laurylacrylat, Myristylacrylat, Cetylacrylat, Stearylacrylat, Oleylacrylat, Behenylacrylat, Hexylmethacrylat, Octylmethacrylat, Nonylmethacrylat, Decylmethacrylat, Laurylmethacrylat, Myristyl-methacrylat, Cetylmethacrylat, Stearylmethacrylat, Oleyl-methacrylat, Behenylmethacrylat oder tert.-Butylcyclohexylacrylat.

Weiterhin eignen sich als Monomere b2) auch N-alkyl- oder N,N-dialkylsubstituierte Carbonsäureamide der Acrylsäure oder der Methacrylsäure, wobei es sich bei den Alkylresten um C₈-C₁₈-Alkyloder Cycloalkylreste handelt, zum Beispiel N-Stearylacrylamid, N-Stearylmethacrylamid, N-Octylacrylamid, N,N-Dioctylacrylamid, N,N-Dioctylmethacrylamid, N-Cetylacrylamid, N-Cetylmethacrylamid, N-Dodecylacrylamid, N-Dodecylmethacrylamid, N-Myristylacrylamid, 2-Ethylhexylacrylamid. Im Falle der N,N-Dialkylamide sind C₈- und C₉-Alkylreste bevorzugt.

Als weitere Monomere B eignen sich Vinylester aliphatischer Carbonsäuren (C₈- bis C₃₀-Carbonsäuren). Bevorzugt finden Vinylester von C₈ bis C₁₈-Carbonsäuren Verwendung, beispielsweise Vinylester der Octan-, Nonan-, Decan-, Undeca-, Laurin-, Tridecan-, Myristin-, Palmitin-, Stearin-, Arachin- oder Behensäure oder der Ölsäure.

Selbstverständlich können auch Mischungen aus zwei oder mehreren Carbonsäureestern, Carbonsäureamiden oder Vinylestern eingesetzt werden, solange die Summe der Anteile dieser Comonomere nicht 40 Mol-% überschreitet.

Als weitere Comonomere C) können folgende copolymerisierbaren Monomere (oder auch Mischungen derselben) verwendet werden (0 - 39 Mol-%, bevorzugt 0 - 20 Mol-%, besonders bevorzugt 0 - 10 Mol-% ).

Monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure Crotonsäure, Fumarsäure, Mesaconsäure und Itaconsäure. Aus dieser Gruppe von Monomeren verwendet man vorzugsweise Acrylsäure, Methacrylsäure, Maleinsäure oder Mischungen der genannten Carbonsäuren. Die monoethylenisch ungesättigten Carbonsäuren können in Form der freien Säure und - soweit vorhanden-der Anhydride oder in partiell oder in vollständig neutralisierter Form bei der Copolymerisation eingesetzt werden. Um diese Monomeren zu neutralisieren, verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetraethylenpentamin.

Weitere geeignete Comonomere C) sind beispielsweise die C₁-C₄-Alkylester, Amide und Nitrile der oben angegebenen Carbonsäuren, z.B. Acrylsäuremethylester, Acrylsäureethylester, Methacrylsäuremethylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, Acryl-amid, Methacrylamid, N,N-Dimethylacrylamid, N-tert.-butylacrylamid, Acrylnitril, Methacrylnitril, Dimethylaminoethylacrylat, Diethyl-aminoethylacrylat, Diethylaminoethylmethacrylat sowie die Salze der zuletzt genannten Monomeren mit Carbonsäuren oder Mineralsäuren sowie die quaternierten Produkte.

Außerdem eignen sich als andere copolymerisierbare Monomere C) Acrylamidoglycolsäure, Vinylsulfonsäure, Allylsulfonsäure, Methallylsulfonsäure, Styrolsulfonsäure, Acrylsäure-(3-sulfopropyl)ester, Methacrylsäure(3-sulfopropyl) ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure.

Weitere geeignete copolymerisierbare Verbindungen C) sind, N-Vinylcaprolactam, N-Vinylimidazol, N-Vinyl-2-methylimidazol, N-Vinyl-4-methylimidazol, Diallylammoniumchlorid, Vinylacetat und Vinylpropionat. Es können auch Mischungen der genannten Monomeren einzusetzen.

Die Herstellung der Copolymerisate erfolgt nach bekannten Verfahren, z.B. der Lösungs-, Fällungs-, oder umgekehrten Suspensionspolymerisation unter Verwendung von Verbindungen, die unter den Polymerisationsbedingungen Radikale bilden.

Die Polymerisationstemperaturen liegen üblicherweise in dem Bereich von 30 bis 200, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Die Copolymeren besitzen K-Werte von mindestens 7 bis 130, vorzugsweise 10 bis 100, besonders bevorzugt 10 - 20. Die K-Werte werden bestimmt nach H. Fikentscher, Cellulose-Chemie, Band 13, 58 bis 64 und 71 bis 74 (1932) in wäßriger Lösung bei 25°C, bei Konzentrationen, die je nach K-Wert-Bereich zwischen 0,1% und 5% liegen.

Die genannten Copolymere eignen sich erfindungsgemäß zum Einsatz als oberflächenaktive Substanzen in Zubereitungen, die als wesentlichen Bestandteil einen oder mehrere wasserunlösliche Stoffe enthalten. Wasserunlösliche Stoffe im Sinne dieser Erfindung sind Stoffe, die in Wasser unter Anwendungsbedingungen überwiegend ungelöst sind. D.h., dass unter Anwendungsbedingungen keine effektive Menge des Stoffes gelöst ist. Demgemäss sind die wasserunlöslichen Stoffe mit Wasser unter Normalbedingungen nicht mischbar, bzw. bilden spontan keine homogene Phase mit Wasser. Wasserunlösliche Stoffe im Sinne dieser Erfindung sind vor allem pharmazeutische oder kosmetische Wirkstoffe, aber auch Wirkstoffe für den Einsatz in Pflanzenschutzmitteln, weiterhin veterinärmedizinische Wirkstoffe, Nahrungsergänzungsmittel, z. B. für diätetische Lebensmittel, Lebenssmittelfarbstoffe oder Zusatzstoffe für die Tierernährung. Die Copolymere können dabei zur Verbesserung der Bioverfügbarkeit schwerlöslicher oder unlöslicher Wirkstoffe dienen, oder als Solubilisatoren in wäßrigem Milieu oder zur Stabilisierung wässriger Dispersionen wie Suspensionen oder Öl-in-Wasser-Emulsionen.

Im Bereich der pharmazeutischen Formulierungen eignen sich die Copolymere für feste oral zu applizierende Arzneiformen, Suppositorien, vaginale Anwendungsformen, Tropfen für transmucosale Anwendung, Augentropfen oder Ohrentropfen, Arzneimittellösungen, pulmonale Anwendungsformen, medizinische Nagellacke, medizinische Haarpflegemittel, parenteral zu verabreichende Arzneiformen, parenterale Ernährungszubereitungen, oder für transdermale Formen zur Verbesserung der Wirkstoffaufnahme durch die Haut. Transdermale Formen können neben Cremes, Salben, Gelen oder Lotionen auch Pflaster wie Nikotinpflaster oder beispielsweise Pflaster zur Verabreichung von Steroidhormonen, Nitroverbindungen, Analgetica, Hydroxycarbonsäuren, Vitamin-A-Säure oder andere Retinoide, Antimykotika, Antiinfectiva, Virostatika, alpha- oder beta-Blocker sein.

Kosmetische Formulierungen sind erfindungsgemäß Mittel für die Hautpflege, die Haarkosmetik, die Nagelpflege oder für die Mundhygiene. Die Copolymere eignen sich beispielsweise zur Solubilisierung oder Stabilisierung von Parfumölen, ätherischen Ölen, Essenzen oder öligen Badezusätzen.

Zubereitungen für den Pflanzenschutz umfassen Pesticide, Herbicide, fungicide oder Insectizide, vor allem auch solche Zubereitungen, die als Spritz- oder Gießbrühen zum Einsatz kommen.

Weiterhin eignen sich die Copolymere im Nahrungsmittelbereich beispielsweise als Schutzkolloide oder Dispergierhilfsmittel für schwerlösliche Lebensmittelfarbstoffe wie beispielsweise Carotinoide.

Ebenso können die Copolymere als Schutzkolloide bei der Emulsions- oder Suspensionspolymerisation oder in Zubereitungen für die Fotografie oder in Tonern eingesetzt werden.

Die Copolymere können in Mengen von 0,05 Gew.-% bis 20 Gew.-%, vorzugsweise 0,1 bis 10 Gew.-%, besonders bevorzugt 0,5 bis 5 Gew.-%, bezogen auf die Gesamtformulierung eingesetzt werden. Die Formulierungen können flüssige, bevorzugt wässrige Formulierungen sein oder auch feste oder halbfeste Formulierungen. Sie können neben den Copolymeren und den wasserunlöslichen Stoffen noch für das jeweilige Anwendungsgebiet übliche Hilfsstoffe in den dafür üblichen Mengen enthalten.

### Beispiele 1 bis 4

Mit den in den nachstehenden Beispielen genannten Polymeren wurde die solubilisierende Wirkung für den Wirkstoff Diazepam getestet. Es wurden 1 Gew.-%ige wäßrige Polymerlösungen eingesetzt.

| Polymer | K-Wert (1%ig in Wasser) | Solubilisierung |
|---|---|---|
| Beispiel 1 N-Vinylpyrrolidon/ Stearylacrylat Copolymer (90 : 10 Gew.-%) | 20,0 | 185 µg/ml Diazepam |
| Beispiel 2 N-Vinylpyrrolidon-Cetylmethacrylat-Copolymer (80 : 20 Gew.-%) | 18,3 | 205 µg/ml Diazepam |
| Beispiel 3 N-Vinylpyrrolidon-Vinylcaprolactam-Dodecylmethacryl-amid-Terpolymer (60 : 20 : 20 Gew.-%) | 10,9 | 200 µg/ml Diazepam |
| Beispiel 4 N-Vinylpyrrolidon-Stearinsäurevinylester-Copolymer (90 : 10 Gew.-%) | 25 | 195 µg/ml Diazepam |

## Patentansprüche

1. Verwendung von Copolymeren aus
A) 60 bis 99 mol-% N-Vinylpyrrolidon, und
B) 1 bis 40 mol-% eines Monomeren ausgewählt aus der Gruppe der
b1) C₈-C₃₀-Alkylester von monoethylenisch ungesättigten C₃-C₈-Carbonsäuren.
b2) N-Alkyl- oder N,N-Dialkyl-substituierten Amide der Acrylsäure oder der Methacrylsäure mit C₈-C₁₈-Alkylresten, oder
b3) der Vinylester von aliphatischen C₈-C₃₀-Carbonsäuren, oder Gemischen dieser Monomeren,
als oberflächenaktive Substanzen in Zubereitungen wasserunlöslicher Stoffe.

2. Verwendung nach Anspruch 1, wobei die Copolymeren 0 bis 39 mol-% weiterer radikalisch copolymerisierbarer Monomere C) enthalten.

3. Verwendung nach Anspruch 1 oder 2, wobei die Zubereitungen als wasserunlösliche Substanzen pharmazeutische Wirkstoffe enthalten.

4. Verwendung nach Anspruch 1 oder 2, wobei die Zubereitungen als wasserunlösliche Substanzen kosmetische Wirkstoffe enthalten.

## Claims

1. The use of copolymers of
A) 60-99 mol% of N-vinylpyrrolidone, and
B) 1-40 mol% of a monomer selected from the group of
b1) C₈-C₃₀-alkyl esters of monoethylenically unsaturated C₃-C₈-carboxylic acids,
b2) N-alkyl- or N,N-dialkyl-substituted amides of acrylic acid or of methacrylic acid with C₈-C₁₈-alkyl radicals, or
b3) vinyl esters of aliphatic C₈-C₃₀-carboxylic acids, or mixtures of these monomers,
as surface-active substances in preparations of water-insoluble substances.

2. The use as claimed in claim 1, wherein the copolymers comprise 0-39 mol% of other free-radical copolymerizable monomers C).

3. The use as claimed in claim 1 or 2, wherein the preparation comprises pharmaceutical agents as water-insoluble substances.

4. The use as claimed in claim 1 or 2, wherein the preparation comprises cosmetic agents as water-insoluble substances.

## Revendications

1. Utilisation de copolymères de
A) 60 à 99 % en mol de N-vinylpyrrolidone, et
B) 1 à 40 % en mol d'un monomère choisi dans le groupe des
b1) esters d'alkyle en C₈-C₃₀ d'acides carboxyliques en C₃-C₈ à insaturation éthylénique,
b2) amides N-alkyl- ou N,N-dialkyl-substituées de l'acide acrylique ou de l'acide méthacrylique avec des restes alkyle en C₈-C₁₈, ou
b3) des esters vinyliques d'acides carboxyliques aliphatiques en C₈-C₃₀,
ou de mélanges de ces monomères,
comme substances tensioactives dans des préparations de substances insolubles dans l'eau.

2. Utilisation selon la revendication 1, dans laquelle les copolymères contiennent 0 à 30 % en mol d'autres monomères C) copolymérisables par voie radicalaire.

3. Utilisation selon la revendication 1 ou 2, dans laquelle les préparations contiennent en tant que substances insolubles dans l'eau des substances à activité pharmaceutique.

4. Utilisation selon la revendication 1 ou 2, dans laquelle les préparations contiennent en tant que substances insolubles dans l'eau des substances à activité cosmétique.
